Europäisches Patentamt

European Patent Office

Office européen des brevets

(19)

(11) **EP 1 149 573 A2**

(12) **EUROPEAN PATENT APPLICATION**

(43) Date of publication:
**31.10.2001 Bulletin 2001/44**

(51) Int Cl.[7]: **A61K 6/083**

(21) Application number: **01109930.6**

(22) Date of filing: **24.04.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.04.2000 JP 2000130713**

(71) Applicant: **KABUSHIKI KAISHA SHOFU Kyoto-shi, Kyoto-fu (JP)**

(72) Inventors:
• **Teramae, Mitsuji, c/oKabushiki Kaisha Shofu Kyoto-shi, Kyoto (JP)**
• **Fujii, Toshihide, c/oKabushiki Kaisha Shofu Kyoto-shi, Kyoto (JP)**
• **Negoro, Noriyuki, c/oKabushiki Kaisha Shofu Kyoto-shi, Kyoto (JP)**

(74) Representative: **Eisenführ, Speiser & Partner Arnulfstrasse 25 80335 München (DE)**

(54) **A dental composite material comprising aggregate**

(57) A dental composite material comprising (A) a polymerizable monomer, (B) a filler comprising at least one heat-treated aggregate of silica and at least one metal oxide other than silica, wherein the aggregate has an average particle size of 0.5-30μm, a refractive index of 1.46-1.65, a pore volume of 0.1-1.0 cc/g, a BET specific surface area of 50-500 m$^2$/g, and a primary particle size of 1-250 nm, and (C) a polymerization initiator. The dental composite material of the present invention can satisfy various kinds of properties required as a dental composite material simultaneously.

**EP 1 149 573 A2**

**Description**

BACKGROUND OF THE INVENTION

The present invention relates to a dental composite material used for teeth-filling restorative materials, materials for crown prosthesis such as inlay, crown and bridge, materials for building up of an abutment, and dental adhesives. More particularly, the present invention relates to a dental composite material having excellent physical strength, particularly excellent wear resistance to toothbrushing and occlusion, proper transparency close to that of natural teeth, excellent surface smoothness of a cured material after polishing, and radiopacity.

In recent years, a dental composite material containing a polymerizable monomer and a filler has been widely used. Such the material is generally referred to as a dental composite resin, and is utilized in various uses such as direct restoration of a tooth defect portion due to dental caries and the like, a crown prosthetic restoration such as by inlay, crown and bridge, the building up of an abutment at a tooth defect portion, and a dental adhesive (composite resin cement). In historical view of the development, the fillers used in such the dental composite material are broadly classified into the following fillers:

One of them is an inorganic filler having an average particle size of around 5-30 μm (macro-filler), which is prepared by grinding a massive inorganic composition such as of quartz and silicate glass. But, a dental composite material containing such the macro-filler is inferior in surface glossiness after polishing, although it is superior in mechanical strength such as flexural strength. In addition, the macro-filler is apt to drop off due to wear by toothbrushing and occlusion and, consequently, the composition has a property of poor wear resistance. Furthermore, in recent years, an inorganic filler, an average particle size of which is adjusted to 0.5-3 μm (submicro-filler), has been used with the development of the grinding technology. However, a dental composite material containing such the submicro-filler still has a property of poor wear resistance since problems such as drop-off of the filler upon application of a wear load remain unsolved, although surface glossiness after polishing has been improved as compared to the dental composite material containing the macro-filler.

Another filler is an inorganic filler of an ultrafine particle having an average particle size of around 0.01-0.05 μm as a primary particle (micro-filler), a representative of which is colloidal silica prepared by a method of combustion-hydrolyzing an organosilane compound. A dental composite material prepared by incorporating such the filler therein is superior in surface glossiness after polishing, but when the packing rate is elevated, then the viscosity of the composite material is increased and, thereby, handling becomes difficult. On the other hand, when the filler content is lowered for this reason, then mechanical strength of the composite material is deteriorated. In addition, there is a shortcoming that the composite material containing the micro-filler turns ashen and opaque due to the light-scattering phenomena (Rayleigh scattering) originated from the ultrafine particle.

Still another filler is an organic compound filler which is prepared by premixing the above ultrafine particle and a polymerizable monomer, and grinding the mixture after curing. Although a rise in the viscosity of the dental composite material can be suppressed and the filler content of the inorganic component can be elevated by using the organic compound filler, as compared to use of a micro-filler alone, mechanical strength of the material is still at a low level and the problem regarding the light-scattering phenomena originated from the ultrafine particle is not solved.

Hitherto, the composite material has been developed by utilizing the above fillers alone or in combination thereof, but various problems as stated above have not been simultaneously solved yet.

In recent years, the development of the filler has further progressed, and new type fillers as described below have been developed. In JP-A 7-196428, there is proposed a filler for a dental composite material in which silicon dioxide and at least one other metal oxide form an amorphous layer, respectively, and which is produced by aggregating silicon dioxide and at least one other metal oxide and heat-treating the aggregate at a temperature lower than a temperature where the oxide crystallizes. According to this invention, there is provided a dental composite material having both of high flexural strength and high smoothness of the polished surface of the cured material, as well as a shade matching with that of natural teeth and radiopacity.

In addition, in JP-A 7-196430, there is proposed a compound filler containing an inorganic particle having an average particle size of less than 1 μm which has been aggregated and heat-treated, and a polymer of a polymerizable monomer. According to this invention, there is provided a compound filler which can provide a dental restorative material having both of high flexural strength and high smoothness of the polished surface of the cured material.

In addition, in JP-A 7-196431, there is proposed a filler for a dental restorative material characterized in that a metal oxide particle having an average particle size of 0.05-1 μm has been aggregated in advance and heat-treated. A dental restorative material using the filler of this invention enables high flexural strength and glossiness of the polished surface to be imparted simultaneously.

In addition, in JP-A 8-12305, there is proposed (C) an inorganic composition containing 60-99 % by weight of (A) a spherical inorganic oxide particle having an average particle size in a range of greater than 0.1 μm and smaller than 1 μm and 40-1 % by weight of (B) an inorganic oxide fine particle having an average particle size of 0.1 μm or

smaller, wherein a volume of a strong aggregate pore having a pore diameter of not less than 0.08 μm is not greater than 0.1 cc/g in (C) an inorganic oxide. Moreover, there is proposed a composite material containing 50-95 % by weight of the above inorganic composition or the inorganic composition in which the surface is treated with a silane coupling agent and 50-5 % by weight of a radical polymerizable monomer. The cured material, which is prepared by curing the composite material of this invention containing the inorganic composition and the radical polymerizable monomer, has excellent surface smoothness, and the surface thereof can be easily polished in a shorter period of time.

In addition, in JP 2510408, there is proposed a non-vitreous micro-particle containing (i) a plural of amorphous micro-regions containing silica, and (ii) a plural of crystalline micro-regions containing a polycrystalline ceramic metal oxide having radiopacity, wherein the above amorphous micro-regions are substantially dispersed uniformly by the crystalline micro-regions and the crystalline micro-regions having a particle size of greater than 0.4 μm or voids. According to this invention, there is provided a micro-particle useful for a dental composite material, and the micro-particle can make a dental composite material radiopaque while imparting to the composite material low visual opacity.

In addition, in JP 2548116, there is proposed a dental composite material having radiopacity, which contains a mixture of (a) a polymerizable resin suitable for oral use and (b) a non-vitreous micro-particle, wherein the non-vitreous micro-particle contains (i) a plural of amorphous micro-regions containing silica and (ii) a plural of crystalline micro-regions containing a polycrystalline ceramic metal oxide having radiopacity, wherein the amorphous micro-regions are substantially dispersed uniformly by the crystalline micro-regions, and wherein the micro-particle does not contain a crystalline micro-region having a particle size of greater than 0.4 μm or a void. According to this invention, there is provided a dental composite material having excellent mechanical strength, transparency, and radiopacity.

In addition, in JP-A 8-143747, there is proposed a compound filler containing 100 parts by weight of (A) a polymer of a polymerizable monomer having two or more vinyl groups in the molecule and 150-2000 parts by weight of (B) a particle having a particle size in a range of 0.01-0.1 μm which contains 1-99 % by weight of silicon dioxide and 99-1 % by weight of at least one oxide of an element belonging to Group II-IV in the Periodic Table (provided that, silicon dioxide is excluded). The filler which is compounded and filled according to the method of this invention (MCF) has a superior color tone and storage stability of a paste over those of an encapsulated filler produced by using benzoyl-$N$,$N$-dimethyl-$p$-toluidine peroxide according to the conventional procedure. Moreover, a composite resin using the filler exhibits the superior mechanical property and transparency over those of the conventional composite resins and, thus, a paste having the good handling property can be provided.

In addition, in JP-A 8-231330, there is proposed a polymerizable dental material containing a methacrylate monomer or an acrylate monomer, a finely-divided inorganic filler and a polymerization catalyst, wherein the inorganic filler contains 5-100% by weight of finely-divided crystalline silicate having a layered structure and 0-95% by weight of an ultrafine glass. According to this invention, there can be provided a dental material which can solve discrepancy of rheological, optical and mechanical goals of the conventional dental materials.

In addition, in JP-A 8-259415, there is proposed a dental material containing A) a spherical $SiO_2$-based particle, B) a mixture of a spherical particle which is prepared by grinding quartz, glass-ceramic and/or glass having a refractive index of 1.50-1.58 and an average particle size of 0.5-5.0 μm, based on a polymerizable ethylenic unsaturated monomer as a binder, a catalyst for low-temperature polymerization, heat-polymerization and/or photopolymerization and an inorganic filler, wherein an amount of the filler is 1-95 % based on that of the dental material, and wherein the component A) the spherical particle in a finally polymerized dental material has A1) $SiO_2$ having the refractive index of about not less than 1.38 to less than 1.50 and an average primary particle size of about 0.04-1.5 μm, A2) a $SiO_2$ core particle covered with an oxide of at least one element belonging to Group I-IV in the Periodic Table having the refractive index of 1.45-1.62, an average particle size of 0.04-1.5 μm, and a covering thickness of about 15-40 nm, and/or A3) a separate particle from either one of the particles A1) and A2), which is additionally covered with a layer of a polymerizable organic binder based on a reaction product of monofunctional or polyfunctional (meth)acrylate and/or isocyanate with methacrylate having hydroxyl groups, wherein the separate particle has a covering layer of a thickness in a range of 5-50 nm, wherein the particle having the polymerizable binder layer has a primary particle size of about 0.04-1.5 μm, and wherein the covered particle has the refractive index in a range of 1.40-1.52. According to the dental material of this invention, there can be prepared a dental material having transparency which is indefinitely adjustable, and suitable polishability and strength.

In addition, in JP 2638349, there is proposed a dental filling material containing a polymerizable monomer and an inorganic filler, wherein the inorganic filler is a mixture of at least two groups of spherical inorganic oxides having different particle sizes in a range of 0.01-30 μm, wherein one group of them contains a spherical inorganic oxide having an average particle size in a range of 1-30 μm, and a ratio of average particle sizes of the spherical inorganic oxides in two groups having close average particle sizes is 2 or more, wherein a group having a larger average particle size is contained at an amount of 50% or more based on the total weight of two groups, and wherein two groups of the inorganic oxide particles have been pre-mixed in their dried state in advance. In the dental filling material prepared by incorporating the inorganic filler defined in this invention into a polymerizable monomer, smaller particles are effectively filled in a gap between larger particles. Therefore, there can be provided a dental filling material which can increase an amount to be filled of the inorganic filler while keeping fluidity high in the paste state, which is low in stickiness and excellent handling such as filing to a defect portion, and which has excellent surface smoothness, low water-absorption, high mechanical strength and a balanced physical property as compared with the conventional dental products.

In addition, in JP-A 9-77626, there is proposed a dental filling material containing 100 parts by weight of (1) a polymerizable monomer, 0.01-10 parts by weight of (2) a polymerization initiator, and 40-400 parts by weight of (3) a compound particle in which a first inorganic oxide is uniformly dispersed in a matrix of vinyl polymer, wherein a difference between the refractive index of a polymer of the polymerizable monomer and that of the compound particle is 0.1 or smaller. According to the dental filling material of this invention, since silica is uniformly dispersed in the vinyl polymer and the compound having an extremely high light-transmittance is contained, there can be provided a cured resin having suitable transparency.

In addition, in JP-A 9-194674, there is proposed a dental filling material containing 100 parts by weight of (a) a polymerizable monomer, 0.01-10 parts by weight of (b) a polymerization initiator, and 40-400 parts by weight of (c) a combination of 1-99 % by weight of a compound polymer particle and 99-1 % by weight of an aggregated particle of the second inorganic oxide having an average particle size of 1-100 μm, wherein the compound polymer is prepared by mechanically kneading a primary particle of the first inorganic oxide having an average particle size of 0.01-1 μm with a polymerizable monomer to polymerize and then grinding, and wherein the aggregated particle contains the primary particle of the inorganic oxide having an average particle size in a range of 0.01-1 μm which contains 1-99 mole % of silica and 99-1 mole % of at least one inorganic oxide of an element belonging to Groups II-IV in the Periodic Table other than silicon. According to the dental filling material of this invention, there can be prepared a paste which perfectly satisfies three requirements of "non-dropping, non-stickiness and good-stretching" and the paste has a suitable thermal stability, since the composition contains the compound polymer particle, the aggregated particle of the second inorganic oxide and/or the primary particle of the first inorganic oxide and, as the result, there can be provided a cured resin having the excellent mechanical property and wear resistance.

In addition, in JP-A 10-67511, there is proposed a silicon dioxide-series fine filler consisting of a porous silicon dioxide glass, wherein the filler has a particle size of 0.5-50 μm, a pore size of 20-120 nm, a pore volume of 20-1000 $mm^3/g$ and the specific surface area according to a BET method of 10-100 $m^2/g$. The dental material in which the filler of this invention is contained exhibits extremely suitable wear strength (wear resistance) and a small shrinkage upon polymerization.

In addition, in JP-A 10-130116, there is proposed a curable composition containing i) a polymerizable monomer, ii) (A) a splintered inorganic particle containing a particle having an average particle size of 1-9 μm and containing 3% by weight or less than of a particle having a particle size of not less than 10 μm, iii) (B) a spherical inorganic particle having an average particle size in a range of 0.1-5 μm, iv) (C) an inorganic fine particle having an average particle size in a range of 0.01-0.1 μm, and v) a polymerization initiator, wherein the curable composition contains (A) the splintered inorganic particle, (B) the spherical inorganic particle and (C) the inorganic fine particle at an amount in a range of 300-1900 parts by weight as a total amount relative to 100 parts by weight of the polymerizable monomer, wherein an amount of (B) the spherical inorganic particle is 50-99 % by weight and an amount of (C) the inorganic fine particle is 50-1 % by weight when a total weight of (B) and (C) is regarded as 100 % by weight, and a ratio of an amount of (A) the splintered inorganic particle relative to a total amount of (B) the spherical inorganic particle and (C) the inorganic fine particle is 0.3-3 as a weight ratio [(A)/{(B)+(C)}], and the polymerization initiator is contained at an amount of 0.01-5 parts by weight relative to 100 parts by weight of the polymerizable monomer. According to the present invention, there can be provided a curable composition having extremely high mechanical strength, particularly extremely high fracture toughness and flexural strength.

In addition, in JP-A 10-306008, there is proposed a dental filling material containing (A) a polymerizable monomer, (B) a polymerization initiator, and (C) an aggregated particle of an inorganic oxide having an average particle size in a range of 1-100 μm, wherein the aggregated particle is composed of a primary particle of an inorganic oxide having an average particle size of 0.01-1 μm which contains 1-99 mole % of silicon dioxide and 99-1 mole % of an inorganic oxide of at least one element belonging to Group II-IV in the Periodic Table. According to this invention, there can be provided a dental filling material having suitable handling, and excellent mechanical strength and wear resistance, and excellent surface glossiness of the cured resin.

The aggregated particles of inorganic oxides shown in the various dental composite material as described above are broadly classified as followings: (1) an aggregated particle prepared by reacting one or more of organometal compounds at the molecular level; and (2) an aggregated particle prepared by reacting an organometal compound onto silica or other metal oxide as a core at the molecular level. However, the number of pores is necessary to be reduced to below the predetermined level by heat treatment in both of the above aggregated particles. In the case where the sufficient number of pores are left, problems such as weakening of particle strength and deterioration of transparency arise. Moreover, in the case of the aggregated particle which is prepared according to the procedure (2), problems such as deterioration of transparency due to its layered structure arise.

As described above, various properties are required to the dental composite materials which have hitherto achieved a high technological innovation. Although the previous dental filling materials had been developed making the greatest point of the surface hardness or flexural strength of a cured material in the physical properties, the properties such as wear resistance to toothbrushing or occlusion and low wearability to opposing natural teeth have been considered important in the course of the recent studies. Also, properties such as surface smoothness after polishing and radiopacity of a cured material have been demanded. In view of aesthetic of the dental composite material, the precise reproduction of light-transmittance, refractivity and scatterability close to those of natural teeth substance are aimed, in addition to a mere white color tone of teeth. Furthermore, in view of handling of a paste prior to curing, a material having suitable stretching, non-stickinesss, and non-dropping properties is desired. There has been no material for the dental composite material, which satisfies such various kinds of properties simultaneously.

## SUMMARY OF THE INVENTION

The present inventors found that the above problems can be solved by incorporating into a dental composite material a filler comprising at least one heat-treated aggregate of silica and at least one metal oxide other than silica, wherein the aggregate has an average particle size of 0.5-30 μm, a refractive index of 1.46-1.65, a pore volume of 0.1-1.0 cc/g, a BET specific surface area of 50-500 $m^2/g$, and a primary particle size of 1-250 nm.

The dental composite material according to the present invention is specifically illustrated below. The present invention is not particularly limited to examples of the composition. As (A) a polymerizable monomer in the present invention, the known monofunctional or polyfunctional polymerizable monomers which are generally used for the dental material may be used. Among them, as the monofunctional polymerizable monomer, hydrocarbon esters of methacrylic acid such as methyl methacrylate, ethyl methacrylate, butyl methacrylate, hexyl methacrylate and the like, as well as acrylates corresponding to above methacrylates are used. Preferably, methyl methacrylate is used.

In addition, as the monofunctional polymerizable monomer having hydroxyl groups, 2-hydroxyethyl methacrylate, 2- or 3-hydroxypropyl methacrylate, 4-hydroxybutyl methacrylate, diethyleneglycol monomethacrylate, dipropyleneglycol monomethacrylate, glycidyl methacrylate, tetrahydrofurfuryl methacrylate, allyl methacrylate and the like, as well as acrylates corresponding to above methacrylates are used. Preferably, 2-hydroxyethyl methacrylate is used.

As silane compounds having methacryloxyalkyl groups, γ-methacryloxypropyltrimethoxysilane, γ-methacryloxypropyltriethoxysilane and the like, as well as acrylates corresponding to above methacrylates are used. Preferably, γ-methacryloxypropyltrimethoxysilane is used.

In addition, as bifunctional monomers of the polyfunctional polymerizable monomer, polymethacrylates of alkane polyol such as ethyleneglycol dimethacrylate, propyleneglycol dimethacrylate and neopentylglycol dimethacrylate, as well as acrylates corresponding to above methacrylates, preferably ethyleneglycol dimethacrylate; polymethacrylates of polyoxyalkane polyol such as diethyleneglycol dimethacrylate, triethyleneglycol dimethacrylate and dipropyleneglycol dimethacrylate, as well as acrylates corresponding to above methacrylates, preferably triethyleneglycol dimethacrylate; bifunctional methacrylates having a urethane linkage prepared by an addition reaction of one mole of diisocyanate compound and two moles of methacrylate containing hydroxyl groups such as 2-hydroxyethyl methacrylate, as well as acrylates corresponding to above methacrylates; and 2,2-bis(4-(2-hydroxy-3-methacryloxypropoxy)phenyl)propane (bis-GMA), 2,2-bis(4-methacryloyloxyphenyl)propane, 2,2-bis(4-methacryloyloxypolyethoxyphenyl)propane (D-2.6E), 2,2-bis(4-methacryloyloxydiethoxyphenyl)propane, 2,2-bis(4-methacryloyloxytetraethoxyphenyl)propane, 2,2-bis(4-methacryloyloxypentaethoxyphenyl)propane and 2,2-bis(4-methacryloyloxydipropoxyphenyl)propane, as well as acrylates corresponding to above methacrylates, but preferably 2,2-bis(4-(2-hydroxy-3-methacryloxypropoxy)phenyl)propane (bis-GMA) is used.

As trifunctional monomers, trimethylolpropane trimethacrylate, trimethylolethane trimethacrylate, pentaerythritol trimethacrylate, trimethylolmethane trimethacrylate and the like, as well as acrylates corresponding to above methacrylates are used. Preferably, trimethylolpropane trimethacrylate is used.

As tetrafunctional monomers, pentaerythritol tetramethacrylate, tetrafunctional urethane methacrylate, and acrylates corresponding to above methacrylates and the like are used. Moreover, penta- or more functional monomers may be used in some cases. Above monofunctional or polyfunctional polymerizable monomers may be used alone or in combination thereof.

Next, (B) the filler containing at least one kind of a heat-treated aggregate of silica and at least one kind of metal oxide other than silica, wherein the aggregate has an average particle size of 0.5-30 μm, a refractive index of 1.46-1.65, a pore volume of 0.1-1.0 cc/g, a BET specific surface area of 50-500 $m^2/g$, and a primary particle size of 1-250 nm is explained in detail.

A method for producing the aggregate in (B) the filler of the present invention is not particularly limited, but a method in which a colloidal solution of silica and a colloidal solution of metal oxide other than silica are spray-dried is suitably used. As the colloidal solution, a commercially available sol solution, a solution of a metal oxide sol which may be prepared by the following method, or the like may be used: After cooling a solution containing 14.2 % by weight of titanium sulfate such that a temperature thereof becomes 10 °C with stirring, 28% aqueous ammonia is added thereto to prepare a slurry of titanate hydrate. Then, the slurry is filtrated and washed with 0.5 % aqueous ammonia to perfectly wash out a sulfate radical and, thereafter, the slurry is filtrated with distilled water to wash out ammonia. Hydrochloric acid is added to the resulting slurry of titanate hydrate to adjust a pH of the slurry to 2 or lower, which is sufficiently stirred to obtain a titanate oxide sol containing 31.7 % by weight of titanium in terms of $TiO_2$. An aggregate having a desired shape and particle size may be prepared by adjusting temperature, humidity, flow rate of gas, gas-liquid ratio, and particle size in the solution upon spray-drying of the colloidal solution. The particle may be prepared as a spherical shape by spray-drying at a temperature of 10-100 °C and a humidity of 13-3 % by volume. Such the aggregate of the spherical shape is preferable, since a paste having the suitable fluidity and a cured material having excellent surface smoothness after polishing may be prepared. The aggregate thus prepared should be heat-treated at 200-1200°C, preferably at 300-1000°C to achieve removal of the remaining solvent and organic component, and the desired pore volume of 0.1-1.0 cc/g and the BET specific surface area of 50-500 m$^2$/g. Although a pore volume and a specific surface area of an aggregate generally tend to be reduced by the treatment at a high temperature over 1000°C, such tendency varies depending on a mixing ratio of silica and metal oxide other than silica and the kind of metal oxide. Therefore, it is necessary that the optimal condition of the heat-treatment is determined by performing a production experiment.

Each aggregate in (B) the filler of the present invention has a pore volume of 0.1-1.0 cc/g, preferably of 0.12-0.88 cc/g, and a BET specific surface area of 50-500 m$^2$/g, preferably of 80-400 m$^2$/g. With the aggregate having such the characteristics, a polymerizable monomer can penetrate into a pore on the surface of the aggregate to form a strong bond at an interface. Therefore, a dental composite material after curing has excellent physical strength and such the property that it can stand a great mechanical stress such as wear due to toothbrushing and occlusion, in addition to excellent flexural strength. In the case where a pore volume and a BET specific surface area of an aggregate are too small, physical strength and wear resistance are inferior. On the other hand, in the case where a pore volume and a BET specific surface area of an aggregate are too large, it becomes difficult to incorporate a larger amount of an aggregate into the polymerizable monomer. As the result, the content of an aggregate become low, leading to inferior physical strength and wear resistance. Moreover, even in the case where an excessive wear stress is applied, only a part of the aggregate of (B) the filler of the present invention is worn out, since the aggregate is a heat-treated aggregate of the ultrafine particle having a primary particle size of 1-250 nm, preferably 5-100 nm. As the result, an amount of the aggregate which may be worn out is minimized and, thereby, the surface aspect of a cured material is always kept in a smooth state. On the other hand, in the case of the conventional inorganic filler of ground glass type, the whole particle is worn out and, thereby, suitable wear resistance is not exhibited and the surface aspect of the cured material after wearing becomes rough, promoting additional wear.

A colloidal solution of silica and a colloidal solution of at least one metal oxide other than silica are used to produce an aggregate in (B) the filler. As at least one metal oxide other than silica, oxides of metal elements such as Al, Ba, Bi, Ca, Ce, Co, Cu, Er, Fe, Hf, Ho, In, La, Mg, Mn, Nd, Ni, Pb, Sb, Sn, Sr, Ta, Ti, Y, Yb, Zn and Zr may be indefinitely used. Although the colloidal solutions of silica and at least one metal oxide other than silica may be mixed in an optional ratio, it is preferable that a mixing ratio of the colloidal solution of at least one metal oxide other than silica is controlled in order to adjust a refractive index of a produced aggregate to 1.46-1.65, which is the refractive index range of a polymerizable monomer generally used in the dental field. Moreover, since the dental composite material of the present invention is used in an oral cavity of a patient, a metal oxide other than silica, used in an aggregate, having the higher safety to the human body is suitably used. As a preferable metal oxide, there are metal oxides of Al, Ba, Ca, Co, Cu, Fe, Hf, La, Mg, Ni, Sr, Ti, Zn and Zr. Furthermore, radiopacity can be imparted to a dental composite material by selecting, as a metal oxide other than silica, an oxide of a metal having a relatively large atomic number. As a metal oxide suitable for imparting radiopacity to a dental composite material, oxides of Ba, La, Sr and Zr may be used. Preferably, an oxide of Sr or Zr may be used. Radiopacity suitable for the dental material is exhibited by incorporating the above metal oxide into an aggregate at an amount of 10 % by weight or greater in terms of an oxide.

In order to obtain aggregates having the different refractive index, a kind and a mixing ratio of at least one metal oxide other than silica should be varied. The adjustment of an average particle size, a pore volume, a pore size distribution or a BET specific surface area can be attained by varying the production conditions of an aggregate, even when a kind and a mixing ratio of at least one metal oxide other than silica, which constitutes the aggregate, are not varied. As described above, two or more kinds of aggregates having different constitutive components or properties may be contained in (B) component (filler) of the present invention and, thereby, additional properties can be imparted to a dental composite material. Particularly, it becomes possible to prepare a dental composite material in the paste condition. Furthermore, transparencies of a dental composite material before and after polymerization can be kept constant by adjusting the refractive indices of these particles such that they have a certain relationship with the refractive indices before and after polymerization of (A) a polymerizable monomer.

Other known fillers generally used in a dental composite material may be contained in (B) the filler of the present invention, in addition to the aggregate. As such the fillers, there are an inorganic filler, an organic filler, an organo-inorganic compound filler and the like. As the inorganic filler, for example, there are silica, aluminum silicate, alumina, titania, zirconia, various glasses (fluoride glass, borosilicate glass, soda-glass, barium glass, barium-aluminium silicate glass, glass containing strontium and zirconium, ceramic-glass, fluoroaluminosilicate glass, and synthetic glass made by a sol-gel method), Aerosil (registered trademark), calcium fluoride, strontium fluoride, calcium carbonate, kaolin, clay, mica, aluminium sulfate, calcium sulfate, barium sulfate, titanium oxide, calcium phosphate, hydroxyapatite, calcium hydroxide, strontium hydroxide, zeolite and the like. As the organic filler, for example, there are poly(methyl methacrylate) (PMMA), poly(ethyl methacrylate), poly(propyl methacrylate), poly(butyl methacrylate), poly(vinyl acetate), polyethyleneglycol, polypropyleneglycol, polyvinylalcohol and the like. Moreover, as the organo-inorganic compound filler, for example, there is a particle prepared by polymerization-covering the surface of the above inorganic filler or the aggregate of the present invention with the above compounds exemplified as (A) the polymerizable monomer, and then grinding it to a proper particle size, a particle prepared by a procedure in which the inorganic filler or the aggregate of the present invention is contained in the polymerizable monomer in advance, and then emulsion polymerization or suspension polymerization of the mixture is conducted.

The surface of the above aggregate and/or filler in (B) the filler is preferably treated with the known titanate coupling agent, aluminate coupling agent or silane coupling agent. As the silane coupling agent, for example, there are γ-methacryloxypropyltrimethoxysilane, γ-methacryloxypropyltriethoxysilane and the like. Preferably, γ-methacryloxypropyltrimethoxysilane is used. The surface treatment of the aggregate and the filler can be conducted with same or different kind of the coupling agent.

As (C) the polymerization initiator for the dental composite material of the present invention, known compounds generally used in the dental material are indefinitely used. The polymerization initiator is broadly classified into a heat-polymerization initiator and a photo initiator. As the heat-polymerization initiator, for example, there are suitably used organic peroxides such as benzoyl peroxide, parachlorobenzoyl peroxide, 2,4-dichlorobenzoyl peroxide, acetyl peroxide, lauroyl peroxide, tertiary-butyl peroxide, cumenhydroperoxide, 2,5-dimethylhexane-2,5-dihydroperoxide, methyl-ethyl-ketone peroxide and tertiary-butylperoxy benzoate, and azo compounds such as azobisisobutylonitrile, methyl azobisisobutyrate and azobiscyano valeric acid.

The polymerization can be conducted at a room temperature by combining the above organic peroxide and an amine compound. As such the amine compound, secondary or tertiary amines in which an amine group is bonded to an aryl group may be preferably used in view of curing promotion. For example, N,N-dimethyl-p-toluidine, N,N-dimethylaniline, N,N-β-hydroxyethylaniline, N,N-di(β-hydroxyethyl)aniline, N,N-di(β-hydroxyethyl)-p-toluidine, N-methylaniline and N-methyl-p-toluidine are preferable.

It is preferable that sulfinates and borates are further combined with a combination of above organic peroxide and the amine compound. As the sulfinate, there are sodium benzenesulfinate, lithium benzenesulfinate, sodium p-toluenesulfinate and the like. And, as the borate, there are sodium, lithium, potassium, magnesium, tetrabutylammonium, and tetramethylammonium salts of trialkylphenylboron or trialkyl(p-fluorophenyl)boron (wherein the alkyl group is an n-buty, n-octyl, n-dodecyl group or the like). Moreover, the organoboron compounds such as tributylborane and a partial oxide of tributylborane which generate a radical by a reaction with oxygen and water may be also used as a polymerization initiator of an organometal-type.

In addition, as the photo initiator, a photo sensitizer which generates a radical by irradiating with the light may be used. As examples of a photo sensitizer to the ultraviolet light, there are benzoin compounds such as benzoin, benzoin methyl ether and benzoin ethyl ether, benzophenone compounds such as acetoinbenzophenone, p-chlorobenzophenone and p-methoxybenzophenone, and thioxanthone compounds such as thioxanthone, 2-chlorothioxanthone, 2-methylthioxanthone, 2-isopropylthioxanthone, 2-methoxythioxanthone, 2-hydroxythioxanthone, 2,4-diethylthioxanthone and 2,4-diisopropylthioxanthone. Moreover, the photo sensitizer which initiates the polymerization by irradiating with the visible light may be suitably used, since the ultraviolet light harmful to the human body is not required. As the above photo sensitizer, there are α-diketones such as benzil, camphorquinone, α-naphtil, acetonaphthone, p,p'-dimethoxybenzil, p,p'-dichlorobenzilacetil, pentanedione, 1,2-phenanthrenquinone, 1,4-phenanthrenquinone, 3,4-phenanthrenquinone, 9,10-phenanthrenquinone and naphthoquinone. Preferably, camphorquinone is used.

In addition, it is preferable to use a photoaccerator in combination with the above photo sensitizer. Particularly, in the case where a tertiary amine is used as the photoaccerator, a compound having an aromatic ring directly substituted with a nitrogen atom is preferably used. As such the photoaccerator, there are tertiary amines such as N,N-dimethylaniline, N,N-diethylaniline, N,N-di-n-butylaniline, N,N-dibenzylaniline, N,N-dimethyl-p-toluidine, N,N-dimethyl-m-toluidine, N,N-diethyl-p-toluidine, p-bromo-N,N-dimethylaniline, m-chloro-N,N-dimethylaniline, p-dimethylaminobenzaldehyde, p-dimethylaminoacetophenone, p-dimethylaminobenzoic acid, ethyl p-dimethylaminobenzoate, p-dimethylaminobenzoic acid amino ester, N,N-dimethylanthranilic acid methyl ester, N,N-dihydroxyethylaniline, N,N-dihydroxyethyl-p-toluidine, p-dimethylaminophenylalcohol, p-dimethylaminostyrene, N,N-dimethyl-3,5-xylidine, 4-dimethylaminopyridine, N,N-dimethyl-α-naphthylamine, N,N-dimethyl-β-naphthylamine, tributylamine, tripropylamine, triethylamine, N-methyldiethanolamine, N-ethyldiethanolamine, N,N-dimethylhexylamine, N,N-dimethyldodecylamine, N,N-dimethylstearylamine, N,N-dimethylaminoethylmethacrylate, N,N-diethylaminoethylmethacrylate and 2,2'-(n-butylimino)diethanol, barbituric acids such as 5-butylbarbituric acid and 1-benzyl-5-phenylbarbituric acid, and tin compounds such as dibutyltin diacetate, dibutyltin dimaleate, dioctyltin dimaleate, dioctyltin dilaurate, dibutyltin dilaurate, dioctyltin diperacetate, dioctyltin *S,S'*-bis(isooctyl mercaptoacetate) and tetramethyl-1,3-diacetoxydistannoxane. The above photo initiator may be used alone or in combination thereof. An amount of the above initiator and that of promoter to be added may be properly selected, but generally it may be selected from a range of 0.1-5 % by weight based on an amount of the polymerizable monomer.

Furthermore, oxycarboxylic acids such as citric acid, malic acid, tartaric acid, glycolic acid, gluconic acid, $\alpha$-oxyisobutyric acid, 2-hydroxypropanoic acid, 3-hydroxypropanoic acid, 3-hydroxybutanoic acid, 4-hydroxybutanoic acid and dimethylolpropioic acid may be effectively added in order to enhance a photopolymerization ability.

In the dental composite material of the present invention, an amount of (A) the polymerizable monomer is 5-95 % by weight and that of (B) the filler is 95-5 % by weight of (B), preferably an amount of (A) is 10-90 % by weight and that of (B) is 90-10 % by weight, when a total amount of (A) and (B) is regarded as 100 % by weight. In the case where the amount of (A) the polymerizable monomer is less than 5 % by weight, it becomes difficult to blend the filler into the polymerizable monomer. On the other hand, in the case where the amount of the polymerizable monomer exceeds 95 % by weight, physical strength of a cured material of a dental composite material becomes less value. Moreover, an amount of an aggregate in (B) the filler is 5 % by weight or more, preferably 10 % by weight or more, when an amount of (B) the filler is regarded as 100 % by weight. In the case where the amount of the aggregate in (B) the filler is less than 5 % by weight, a dental composite material can not satisfy excellent physical property, wear resistance, surface smoothness, radiopacity, suitable transparency, excellent paste handling simultaneously. An amount of (C) the polymerization initiator is 0.01-10 % by weight. In the case where an amount of (C) the polymerization initiator is more than 10 % by weight, there arise various shortcomings such as deterioration of the storage stability and shortening of working period after the dental composite material is removed from a vessel.

The dental composite material of the present invention, if necessary, may contain various known additives such as polymerization inhibitor, coloring agent, anti-discoloring agent, fluorescent agent, ultraviolet absorbing agent and antimicrobial agent.

A packing type of the dental composite material of the present invention is not particularly limited, but it may be a one-package type, a two-package type and the like depending upon a kind of the polymerization initiator or application procedure, and it may be appropriately selected depending upon use.

The following Examples further illustrate the present invention in more detail, but the present invention is not limited thereto. The measurement of properties of the dental composition of the present invention was performed as follows.

Measurement of average particle size

The measurement of an average particle size was performed with a laser diffraction particle size measuring unit ("Microtrack SPA" manufactured by Nikkiso Inc.).

Measurement of refractive index

A filler and a standard refractive solution (manufactured by CARGILLE Inc.) were mixed in an equal weight to prepare a slurry, and transparency of the slurry was visually confirmed. The refractive index of the standard refractive solution which was observed most transparent was regarded as the refractive index of the filler.

Measurement of pore volume and surface area with BET method

The measurement of a pore volume and a surface area with BET method was performed according to an $N_2$ gas adsorption method with a full automated gas adsorbing unit.

Measurement of flexural strength

A dental composition to be tested was packed in a stainless steel mold (25 X 2 X 2 mm: rectangular parallelepiped-shape). Thereafter, glass covers were placed on both sides of the mold and pressed by a glass plate, then the dental composition was cured by irradiating the front and back sides thereof with the light for 3 minutes each with a powered photopolymerization activator (Solidilight: manufactured by Shofu Inc.). After curing, the cured material was removed from the mold and was immersed in water at 37°C for 24 hours.

The specimen mounted parallel with 20 mm between centers, and flexural strength test equipment and apparatus to provide a constant cross head speed of 1 mm/min with an Instron universal tester (Instron 5567, manufactured by Instron Inc.).

Measurement of toothbrushing wear

Four dental compositions to be tested were packed in stainless steel molds (rectangular mold of 15 × 15 × 2.6 mm and 25 × 15 × 2.6 mm). Thereafter, glass covers were placed on both sides of each mold and pressed by a glass plate, then the dental composition was cured by irradiating the front and back sides thereof with the light for 3 minutes each with a powered photopolymerization activator (Solidilight: manufactured by Shofu Inc.). After curing, the cured material was removed from the mold, and polished successively with a sandpaper of #600 and #1200 to adjust the thickness thereof to 2.5 mm. Then, the surface of the cured material was polished by buffing, and kept in an incubator at 37°C for 24 hours to prepare a test sample. The test sample was mounted on a toothbrushing wear test machine (manufactured by Shofu, Inc.) to perform a toothbrushing wear test of 30000 cycles using a toothbrush (Perio H: manufactured by Sunstar Inc.) and a toothpaste (White: manufactured by Sunstar Inc.). The weight loss (wt %) was calculated from following formula:

(a weight loss of the specimen/ the weight of the specimen before wear) × 100.

Additionally, the test was performed with four samples and the average of the four measurements was evaluated.

Measurement of transparency: Contrast ratio

A dental composition to be tested was packed in a stainless steel mold ($\phi$ 15×1 mm). Thereafter, glass covers were placed on both sides of the mold and were pressed by a glass plate, then the dental composition was cured by irradiating the front and back sides thereof with the light for 3 minutes each with a powered photopolymerization activator (Solidilight: manufactured by Shofu Inc.). After curing, colorimetry of the cured material was performed with a spectrophotometer (CM-2002: manufactured by Minolta Inc.). Transparency of the test sample is evaluated from a contrast ratio as C value: C value=$Y_B/Y_W$ (wherein, $Y_w$ is Y value of colorimetry of the test sample placed on a white board, and $Y_B$ is Y value of colorimetry of the test sample placed on a black board). As the C value approaches zero closer, the material is more transparent and, on the other hand, as the value approaches one closer, the material is more opaque.

Polishability test: Surface roughness

A dental composite material to be tested was packed in a stainless steel mold ($\phi$ 15×1 mm). Thereafter, glass covers were placed on both sides of the mold and pressed by a glass plate, then the dental composite material was cured by irradiating the front and back sides thereof with the light for 3 minutes each with a powered photopolymerization activator (Solidilight: manufactured by Shofu Inc.). After curing, the cured material was removed from the mold, polished successively with a sandpaper of #600 and #1200, and polished by buffing. A centerline average roughness Ra of the prepared sample was measured with a surface roughness-measuring unit (Surfcorder SE-30H: manufactured by Kosaka Laboratory Ltd.).

Measurement of Radiopacity

A dental composition to be tested was packed in a stainless steel mold ($\phi$ 15×2 mm). Thereafter, glass covers were placed on both sides of the mold and pressed by a glass plate, then the dental composition was cured by irradiating the front and back sides thereof with the light for 3 minutes each with a powered photopolymerization activator (Solidilight: manufactured by Shofu Inc.). After curing, the cured material was removed from the mold, the cured material was roentgenographed on a dental X-ray film with an X-ray unit (DCX100N: manufactured by Asahi-Roentgen Industry Ltd.). At the same time, an aluminium plate having a thickness same as that of the cured material was roentgenographed, and radiopacity of the test sample was evaluated. In the case where radiopacity of the sample (2 mm thickness) is equal to that of the aluminium plate (2 mm thickness), it was estimated as 100 %.

Blending of polymerizable monomer and polymerization initiator

Seventy parts by weight of di(methacryloxyethyl)trimethylhexamethylene diurethane (UDMA), 30 parts by weight of triethyleneglycol dimethacrylate (3G), 0.2 parts by weight of camphorquinone, 2 parts by weight of dimethylaminoethyl methacrylate, and 300 ppm of butylated hydroxytoluene (BHT) were mixed to prepare a binder resin (R) for a dental composite material. The refractive indices of the binder resins before and after the polymerization with an Abbe refractometer were measured as 1.49 and 1.51, respectively.

Example 1

A silica sol having an average particle size of 15 nm (Cataloid: manufactured by Catalysts & Chemicals Industry Co., Ltd.) and a zirconia sol having an average particle size of 23 nm (manufactured by Daiichi rare metal Co., Ltd.) were mixed so that a weight ratio of $ZrO_2$ in an aggregate became 7 % by weight, which was subjected to the spray-drying process and heat-treatment to prepare an spherical aggregate (F1). The aggregate had a particle size of 3.1 μm, a refractive index of 1.49, a pore volume of 0.576 cc/g, and a BET specific surface area of 155 $m^2$/g. After the surface of the prepared aggregate was treated with γ-methacryloxypropyltrimethoxysilane, 55 parts by weight of the aggregate (F1), 9 parts by weight of Aerosil R-972 (hydrophobic ultrafine particle silica: average particle size of 16 nm), and 36 parts by weight of the binder resin (R) were kneaded in a double-planetary mixer (DP-0.3 type) while deaerating under the reduced pressure condition to prepare the dental composite material of the present invention.

Example 2

A silica sol having an average particle size of 15 nm (Cataloid: manufactured by Catalysts & Chemicals Industry Co., Ltd.) and a zirconia sol having an average particle size of 23 nm (manufactured by Daiichi rare metal Co., Ltd.) were mixed so that a weight ratio of $ZrO_2$ in an aggregate became 15 % by weight, which was subjected to the spray-drying process and heat-treatment to prepare an spherical aggregate (F2). The aggregate had an average particle size of 4.0 μm, a refractive index of 1.52, a pore volume of 0.277 cc/g, and a BET specific surface area of 218 $m^2$/g.
According to the same manner as that of Example 1 except that the above aggregate was used.

Example 3

According to the same manner as that of Example 1 except that 30 parts by weight of the aggregate of Example 1 (F1) and 25 parts by weight of the aggregate of Example 2 (F2) were mixed, the dental composite material of the present invention was prepared.

Comparative Example 1

A barium-aluminium borosilicate glass (refractive index of 1.570) which is generally used in the dental composite material was ground to an average particle size of 10.4 μm using a vibration mill to prepare an inorganic filler (F3). According to the same manner as that of Example 1 except that the above inorganic filler was used, the dental composite material of Comparative Example was prepared.

Comparative Example 2

A silicate glass (refractive index of 1.460) which is generally used in the dental composite material was ground to an average particle size of 1.4 μm using a vibration mill to prepare an inorganic filler (F4). According to the same manner as that of Example 1 except that the above inorganic filler was used, the dental composite material of Comparative Example was prepared.

Comparative Example 3

Fifty parts by weight of Aerosil OX-50 (ultrafine particle silica: average particle size of 40 nm) which had been surface-treated with γ-methacryloxypropyltrimethoxysilane, 25 parts by weight of di(methacryloxyethyl)trimethylhexamethylene diurethane (UDMA), 25 parts by weight of neopentylglycol dimethacrylate (NPG) and 0.1 parts by weight of benzoylperoxide (BPO) were mixed in a pressurized type-kneader and heated to 120°C to prepare a massive polymer. The above polymer was ground to an average particle size of 22.3 μm with a vibration mill to prepare an organo-inorganic compound filler (F5). According to the same manner as that of Example 1 except that the above organo-inorganic compound filler was used, the dental composite material of Comparative Example was prepared.

Table 1

| | Flexural Strength (MPa) | Amount of toothbrushing wear (% by volume) | Transparency (Contrast Ratio) | | Surface Roughness (Ra) ($\mu$ m) | Radiopacity (%) |
|---|---|---|---|---|---|---|
| | | | Before polymerization | After polymerization | | |
| Example 1 | 110 | 0.445 | 0.15 | 0.24 | 0.023 | 75 |
| Example 2 | 113 | 0.454 | 0.25 | 0.15 | 0.025 | 125 |
| Example 3 | 115 | 0.449 | 0.19 | 0.20 | 0.025 | 100 |
| Comparative Example 1 | 106 | 0.681 | 0.85 | 0.71 | 0.076 | 175 |
| Comparative Example 2 | 113 | 1.944 | 0.60 | 0.71 | 0.059 | 25 |
| Comparative Example 3 | 76 | 0.522 | 0.26 | 0.29 | 0.052 | $\leqq$25 |

The dental composite material of the present invention has excellent physical strength, particularly excellent wear resistance to toothbrushing and occlusion, suitable transparency close to that of natural teeth, surface smoothness after polishing a cured material, and radiopacity. The dental composite material may be used as a filling restorative material of teeth substance, materials for crown prosthesis such as inlay, crown and bridge, materials for building up of an abutment, dental adhesives and the like.

**Claims**

1.  A dental composite material comprising

    (A) a polymerizable monomer,
    (B) a filler comprising at least one heat-treated aggregate of silica and at least one metal oxide other than silica, wherein the aggregate has an average particle size of 0.5-30 $\mu$m, a refractive index of 1.46-1.65, a pore volume of 0.1-1.0 cc/g, a BET specific surface area of 50-500 $m^2$/g, and a primary particle size of 1-250 nm, and
    (C) a polymerization initiator.

2.  The dental composite material according to claim 1, wherein said aggregate is prepared by spray-drying a colloidal solution of silica and a colloidal solution of at least one metal oxide other than silica.

3.  The dental composite material according to claim 1 or 2, wherein said aggregate is spherical.

4.  The dental composite material according to any one of claims 1-3, wherein said aggregate contains at least one metal oxide other than silica at an amount of not less than 10 % by weight in terms of an oxide.

5.  The dental composite material according to any one of claims 1-4, wherein said aggregate is a mixture of two or more kinds of aggregates having the different constituents and properties.